# EUROPEAN PATENT APPLICATION

(11) **EP 1 891 883 A1**
(43) Date of publication of application: **27.02.2008**
(21) Application number: 06017594.0
(22) Date of filing: 23.08.2006
(51) Int. Cl.: A61B 1/01, A61B 1/05

(54) **Disposable two-piece endoscope**

(71) Applicant: Chung Shan Institute of Science and Technology, Longtan Township T'ao yuan (TW)
(72) Inventor: Huang, Ker Jer, Longtan Township Taoyuan County (TW); Kang, Der Ren, Da-an District Taipei City (TW); Weng, Ping Kuo, Longtan Township Taoyuan County (TW); Kuo, Wei Wu, Hsinchu City (TW); Wu, Hsien Ming, Longtan Township Taoyuan County (TW)
(74) Representative: Kador & Partner

(57) **Abstract**

A disposable two-piece endoscope for examining human organs includes an image capturing and transmission device and a cable which may be disconnected. The image capturing and transmission device includes a body, luminous devices, an optical image capturing device, a radio transmission device and an internal power supply. The cable can transmit external electric power and signals. The luminous device is located on the front end of the body to project the human organs. The optical image capturing device captures organ images. The radio transmission device transmits the organ images outside the human body. The internal power supply provides electric power for signal transmission after the cable is separated. After the disposable two-piece endoscope has finished examination of the gullet and stomach, the cable and the image capturing and transmission device may be separated, and the mage capturing and transmission device can enter the intestine to continue examination. Thus the invention can perform examination at one time for the digestive system including the gullet, stomach and intestine.

## Description

### FIELD OF THE INVENTION

The present invention relates to an endoscopic system and particularly to a disposable two-piece endoscope to capture medical images in people's gullet, stomach and small intestine in one examination.

### BACKGROUND OF THE INVENTION

The endoscope is widely used in medical practices to examine medical images of organs or perform surgical operation in a small area. The conventional endoscope usually employs an optical fiber system to capture tissue images by penetrating deeply into hollow organs of a human body (such as stomach, large intestine and trachea) to facilitate determination of the sources and developing conditions of illness. Light is transmitted through an optical fiber cable from a light source to project on the organ tissues. Images are transmitted back through the optical fiber cable to form images on an image sensor. The images are processed by a circuit and displayed on a screen. The optical fiber cable has to include many optical fibers to generate enough pixels. Such optical fiber endoscope is expensive and complex. Fabrication is difficult, and maintenance is not easy. As the optical fiber endoscope is expensive, it has to be used repeatedly. Cross infection among patients is prone to occur if sterilization is not being done properly.

Another problem for the optical fiber endoscope is that the flexible hose is too large and often inflicts a great pain to the patient. Hence many patients are reluctant to take stomach examination that involves the endoscope. Moreover, the present optical fiber endoscope for digestive track can examine only gullet, stomach and large intestine. For the small intestine that has total length of six meters, the optical fiber endoscope can reach only the first ninety centimeters. Hence most of the small intestine cannot be examined.

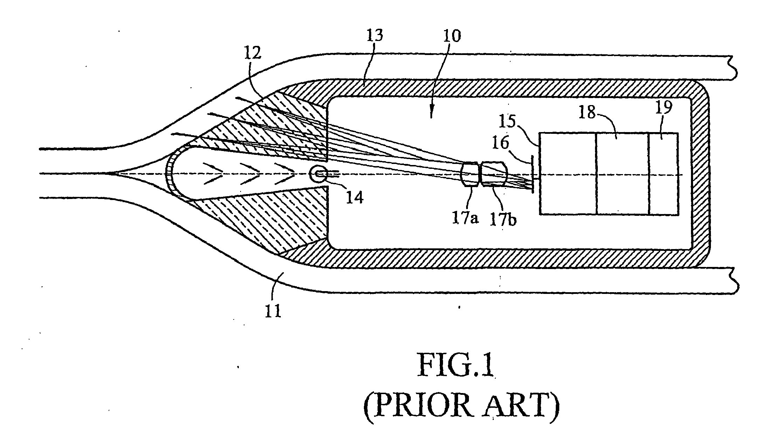

In order to resolve the problem of examining the small intestine, Referring to FIG. 1, Given image corporation (an Israeli company) develops a vivo video camera system disclosed in U.S. patent No. 5,604,531. It can transmit image data by radio to facilitate examination of the inner wall of the small intestine.

It is a radio capsule endoscope 10 including a transparent optical front cover 12 and an opaque capsule shell 13 that are compatible to human body After swallowed by a patient, the digestive tract 11 of the patient is adjacent to the transparent optical front cover 12. A light emitting diode (LED) 14 in the endoscope emits light to pass through the transparent optical front cover 12 and project on the inner wall of the digestive tract 11. Images are transmitted back through the transparent optical front cover 12, an image forming front lens 17a and an image forming rear lens 17b to form the images on a charge-coupled device (CCD) 16. A CCD actuator 17 drives the CCD 16 and sends the image signals to a radio transmitter 18 for transmission. An antenna is located outside the patient to capture the image signals and send the signals to a reception system. After processed by a circuit, the images are stored or displayed on a display device to be interpreted by doctors. A power supply module 19 is included to provide electric power for the operation of the entire capsule endoscope.

The radio capsule endoscope is very helpful for examining the medical images of the small intestine. However, the direction of the lens in the capsule endoscope cannot be controlled. Hence it is not suitable to examine the gullet and stomach. As the number of patients suffering from the small intestine illness is much smaller than those suffering from the stomach illness, application of the capsule endoscope is limited.

### SUMMARY OF THE INVENTION

In view of the aforesaid problems, the primary object of the present invention is to provide a disposable two-piece endoscope that can examine medical images of people's gullet, stomach and small intestine in one time to overcome the disadvantages of the conventional techniques, and offer a great benefit to patients who suffer from the illness of digestive tract.

The disposable two-piece endoscope according to the invention aims to examine human organs. It includes an image capturing and transmission device and a cable. The image capturing and transmission device includes a body, luminous devices, an optical image capturing device, a radio transmission device and an internal power supply. The luminous devices are located on the front end of the body to provide light signals. The optical image capturing device is located behind the luminous devices to convert the light signals to electric signals. The radio transmission device has a circuit connecting to the optical image capturing device to transmit the electric signals of the organs outside the human body. The internal power supply is connected to all the rest devices to provide electric power required. The cable adopts a disconnection structure and is connected to a tail end of the image capturing and transmission device to transmit external electric power and the electric signals.

In addition, when the disposable two-piece endoscope of the invention is used in examining patient's gullet and stomach, transmission of the electric signals of the images is performed through a wired circuit. Hence the medical image data can be displayed in a greater brightness and at a higher speed. For examining the inner wall of the small intestine, the electric signals of the images are transmitted by radio.

The foregoing, as well as additional objects, features and advantages of the invention will be more readily apparent from the following detailed description, which proceeds with reference to the accompanying drawings.

### BRIEF DESCRIPTION OF THE DRAWINGS

FIG. 1 is a schematic view of a conventional capsule endoscope;
FIG. 2 is a schematic view of an embodiment of the disposable two-piece endoscope of the invention;
FIG. 3A is a schematic view of an embodiment of the disconnection mechanism of the invention;
FIG. 3B is a schematic view of the cross section of the steel ball of an embodiment of the disconnection mechanism of the invention;
FIG. 3C is a schematic view of the cross section of the flexible hose of an embodiment of the disconnection mechanism of the invention; and
FIG. 4 is a schematic view of the cross section of the flexible hose of the invention after disconnected.

### DESCRIPTION OF THE PREFERRED EMBODIMENTS

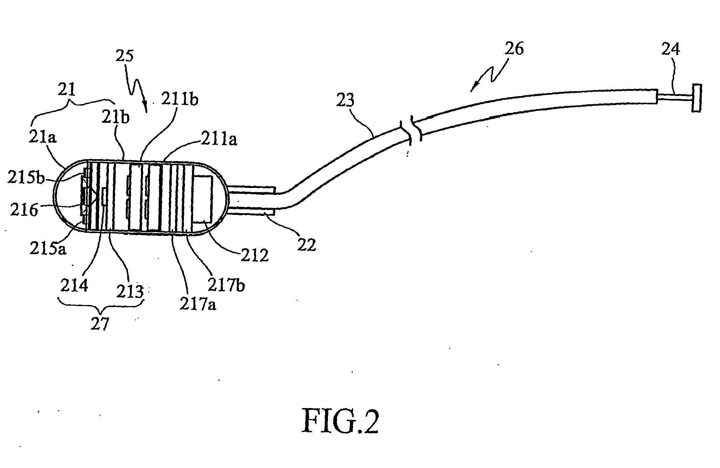

Referring to FIG. 2, the disposable two-piece endoscope according to the invention includes an image capturing and transmission device 25 and a cable 26. The image capturing and transmission device 25 includes a body 21, luminous devices 215a and 215b, an optical image capturing device 27, a radio transmission device 212 and internal power supply 211a and 211b. The body 21 is made from plastics compatible to human body, and includes a transparent front window 21a and a shell 21b. The shell 2 1 b has a diameter smaller than 11mm. The optical image capturing device 27 is located in front of the image capturing and transmission device 25. In this embodiment, the luminous devices 215a and 215b are light emitting diodes (LED). The optical image capturing device 27 includes a complementary metal oxide semiconductor (CMOS) image sensor 214 and an actuation circuit board 213. In front of the image sensor 214, there are the luminous devices 215a and 215b, and an image forming lens assembly 216. Light is emitted from the luminous devices 215a and 215b, passes though the transparent front window 21a, and projects on organs of a human body (such as gullet, stomach and intestine). Images are reflected through the transparent front window 21 a and the image forming lens assembly 216 to form images on the image sensor 214. The image sensor 214 converts the light signals of the images to electric signals through the actuation circuit board 213. The electric signals may be transmitted through two channels. One is through a wired power supply and signal transmission means, with the electric signals transmitted through circuit boards 217a and 217b, a disconnection mechanism 22 and a flexible hose 23 compatible to human body to an external circuit (not shown in the drawing) to be processed and stored or displayed on a display device. This approach is employed when the disposable two-piece endoscope is used to examine the gullet and stomach of the human body. Another channel is radio transmission adopted when the two-piece endoscope is used to examine the intestine of the human body. The electric signals are sent to the radio transmission device 212 (such as an antenna) for transmission. The radio signals pass through the human body and are received by another antenna of a receiver outside the human body, and are processed and stored in the receiver or displayed on a display device.

The internal power supply 211a and 211b (such as batteries) located in the image capturing and transmission device 25 provide electric power to the luminous devices 215a and 215b, image sensor 214, radio transmission device 212 and circuits when the disposable two-piece endoscope is used to examine the intestine of the human body and is disconnected from the power supply of the body 21. As the internal power supply 211a and 211b are used only when the image capturing and transmission device 25 is separated from the disposable two-piece endoscope, electric power requirement is less than the radio capsule endoscope 10, hence the sizes may be made smaller to make swallowing by the people easier.

The cable 26 adopts a disconnection structure, and may be severed by a mechanical force or electric burning. In this embodiment, the cable 26 includes the disconnection mechanism 22, the flexible hose 23 compatible to human body and a pushbutton 24. It is connected to the image capturing and transmission device 25 through the disconnection mechanism 22. When the disposable two-piece endoscope is used to examine human stomach, it is controlled through a wire. The power cord and signal line are connected to the circuit of the image capturing and transmission device 25 through the flexible hose 23 and the disconnection mechanism 22.

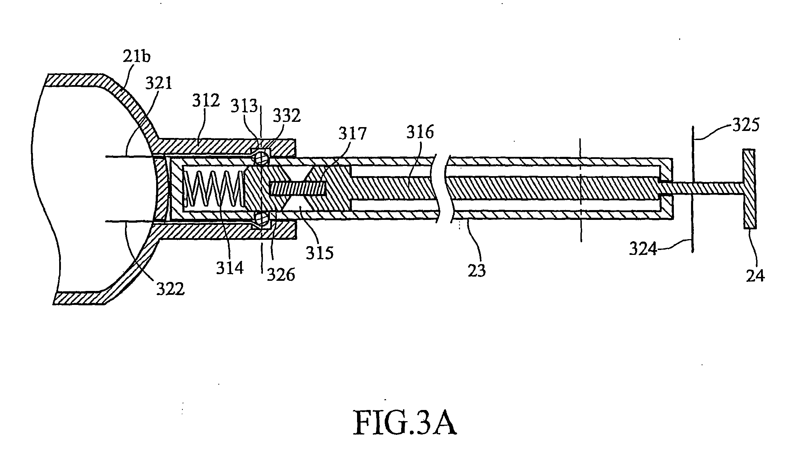

Refer to FIG. 3A for an enlarged sectional view of the cable 26 (not shown in the proportional relationship). The flexible hose 23 contains the disconnection mechanism and is coupled with a disconnection shell 312 extended from the shell 21b on the tail end of the image capturing and transmission device 25. The disconnection mechanism includes a steel ball 332 extending outside the outer surface of the flexible hose 23 to ram an elastic reed 313 into a steel ball trough formed on the disconnection shell 312 so that the flexible hose 23 and the disconnection shell 312 can be coupled together without loosening off. The steel ball 332 is coupled tightly with the elastic reed 313. The elastic reed 313 also is connected to signal lines 321 and 322 located in the image capturing and transmission device 25. The steel ball 332 further is connected to other signals lines 324 and 325 located in the flexible hose 23 to connect to external circuits so that external electric power can be transmitted to the image capturing and transmission device 25 for operation use. And the image data obtained by the image capturing and transmission device 25 can be sent out for storing or displaying outside through the cable 26.

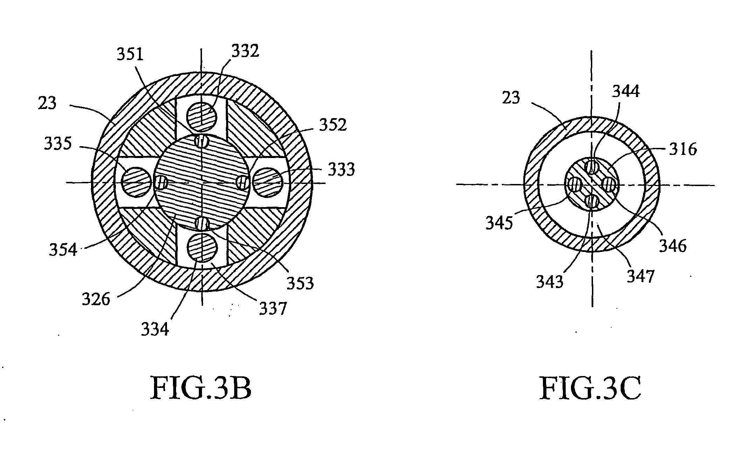

Refer to FIG. 3B for the cross section of the cable 26 of FIG. 3A where the steel ball 332 is located. Steel balls 332 and 334 are connected to power cords 351 and 353, and steel balls 333 and 335 are connected to signal lines 352 and 354. The power cords 351 and 353, and the signal lines 352 and 354 are embedded in an axle 316 which is made from a tough plastics. The steel ball 334 is located in a steel ball trough 337. All elements are encased in the disconnection shell 312.

Refer to FIG. 3C for the cross section of the flexible hose 23. Power cords 344 and 343, and signal lines 345 and 346 are embedded in the axle 316 which is surrounded by a gap 347, then is encased by the flexible hose 23. When the disposable two-piece endoscope is operated in the gullet or stomach of the human body, the required electric power of all elements are supplied externally through the cable 26 in the flexible hose 23, including the luminous devices 215a and 215b, CMOS image sensor 214, radio transmitter and circuits.

By means of the approach set forth, there is no limitation of power supply. Hence a light source of a greater brightness may be provided. Moreover, image signals are transmitted through the cable. Image quality and picture frame number per second can match those achieved by the conventional stomach endoscope. As the image signals are transmitted through a plurality of electric wires, and the flexible hose is pliable, the cable can be made smaller than the optical fiber cable of the conventional endoscope. Moreover, since the cable is pliable and flexible, it can be bent at a smaller curvature radius. By contrast, the optical fiber cable of the conventional endoscope does not have a desired flexibility, and has to be bent at a greater curvature radius, hence is more difficult to use.

After the disposable two-piece endoscope has finished examination of the stomach, the doctor can depress the pushbutton 24 to push the axle 316. The axle 316 moves a ramming member 326 forwards to compress a spring 314. The steel ball 332 is attracted by a strong magnet 317 into another steel ball trough 315 without latching the disconnection shell 312. Then the flexible hose 23 may be separated from the disconnection shell 312.

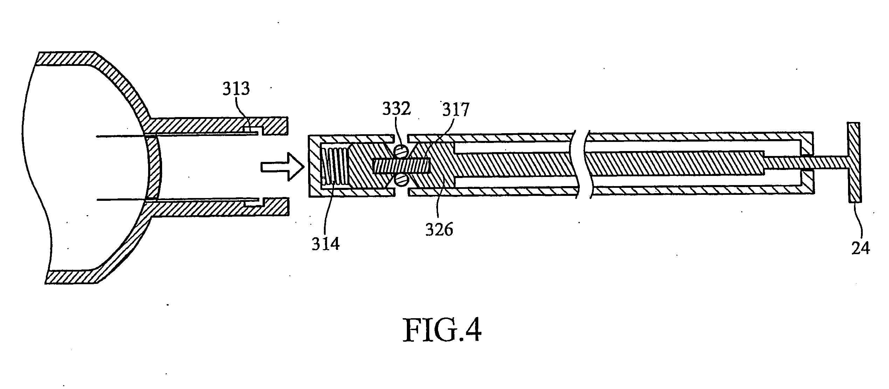

Refer to FIG. 4 for a condition in which the flexible hose 23 is separated from the image capturing and transmission device 25. The pushbutton 24 is depressed, the axle 316 and the ramming member 336 are moved forwards, the spring 314 is compressed, the elastic reed 313 is straightened again, the steel ball 332 is attracted by the strong magnet 317 and disengaged. Hence the image capturing and transmission device 25 can enter the intestine smoothly. In the image capturing and transmission device 25, there is an information switch. When the steel ball 332 is loosened, the internal power supply 211a and 211b in the image capturing and transmission device 25 are activated to provide electric power to the image capturing and transmission device 25. The image capturing and transmission device 25 functions as a general radio capsule endoscope. Hence the two-piece capsule endoscope has the advantages of the optical fiber stomach endoscope and the radio capsule endoscope, and can perform a completed examination for the digestive system.

While the preferred embodiments of the invention have been set forth for the purpose of disclosure, modifications of the disclosed embodiments of the invention as well as other embodiments thereof may occur to those skilled in the art. Accordingly, the appended claims are intended to cover all embodiments which do not depart from the spirit and scope of the invention.

## Claims

1. A disposable two-piece endoscope for examining organs of human body ( 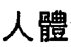 ), comprising:
an image capturing and transmission device including:
a body;
a luminous device located in the body to provide a light signal to project the organs of the human body;
an optical image capturing device located behind the luminous device to convert the light signal to an electric signal;
a radio transmitter having a circuit connecting to the optical image capturing device to transmit the electric signal outside the human body; and
an internal power supply located in the body having another circuit connecting to the body, the luminous device, the optical image capturing device, and the radio transmitter to provide electric power required; and
a cable connecting to a tail end of the image capturing and transmission device to channel an external power supply and transmit the electric signal, and having a disconnection structure to be disconnected from the image capturing and transmission device.

2. The disposable two-piece endoscope of claim 1, wherein the body has a transparent front window and a shell, the transparent front window being located in front of the shell for housing the luminous device.

3. The disposable two-piece endoscope of claim 1, wherein the body is made from a material compatible to the human body.

4. The disposable two-piece endoscope of claim 1, wherein the cable includes two or more power cords and two or more signal lines to transmit respectively electric power and the electric signals.

5. The disposable two-piece endoscope of claim 1, wherein the cable further includes a disconnection mechanism which is coupled with the image capturing and transmission device to control connection and disconnection between the cable and the image capturing and transmission device.

6. The disposable two-piece endoscope of claim 5, wherein the cable further includes a flexible hose which is made from a material compatible to the human body and coupled with a rear end of the disconnection mechanism.

7. The disposable two-piece endoscope of claim 6, wherein the flexible hose includes a steel ball, the disconnection mechanism including a steel ball trough and an elastic reed, the elastic reed having one end fastened to one side of the steel ball trough, the flexible hose being latched in the steel ball trough by pressing the steel ball on the elastic reed, the elastic reed being connected electrically to the image capturing and transmission device through the flexible hose.

8. The disposable two-piece endoscope of claim 5, wherein the cable further includes a pushbutton controlled by human hands to loosen or fasten the disconnection mechanism.

9. The disposable two-piece endoscope of claim 8, wherein the cable further includes an axle which is connected to the pushbutton and movable by the pushbutton to move the steel ball away from the steel ball trough to disconnect the flexible hose from the disconnection mechanism.

10. The disposable two-piece endoscope of claim 1, wherein the cable is severed by a mechanical force or electric burning.

11. The disposable two-piece endoscope of claim 1, wherein the shell of the image capturing and transmission device has a diameter smaller than 11 millimeters.

12. The disposable two-piece endoscope of claim 1, wherein the luminous element is a light emitting diode.

13. The disposable two-piece endoscope of claim 1, wherein the optical image capturing device includes an image sensor to receive the light signal and an actuation circuit board to convert the light signal to the electric signal.

14. The disposable two-piece endoscope of claim 13, wherein the image sensor is a complementary metal oxide semiconductor (CMOS) image sensor.
